# FASCICULE DE BREVET EUROPEEN

(11) **EP 1 925 271 B1**
(45) Date de publication et mention de la délivrance du brevet: **19.08.2009**
(21) Numéro de dépôt: 06024186.6
(22) Date de dépôt: 22.11.2006
(51) Int. Cl.: A61F 2/30, A61F 2/44, A61F 2/46, A61F 2/00

(54) **Implant intervertébral**
Zwischenwirbelimplantat
Intervertebral implant

(43) Date de publication de la demande: 28.05.2008
(73) Titulaire: Eden Spine Europe SA, 1207 Genève (CH)
(72) Inventeur: Ben-Mokhtar, Mourad, 1207 Genève (CH); Rogeau, Dominique, 08034 Barcelone (ES)
(74) Mandataire: Micheli & Cie SA

(56) Documents cités:
- WO-A-20/05087143
- WO-A-20/06066228
- WO-A-20/06079356
- DE-C1- 19 710 392
- US-A1- 2006 241 761

## Description

La présente invention concerne un implant intervertébral, destiné à maintenir un espace souhaité entre deux corps vertébraux d'une colonne vertébrale. Un tel implant est également connu sous le nom de « cage de fusion intersomatique ». Il sert à traiter les pathologies du disque intervertébral.

Une cage de fusion intersomatique est généralement placée à l'intérieur d'un disque intervertébral dont on a réalisé préalablement une discectomie partielle. La cage de fusion intersomatique rétablit la hauteur du disque intervertébral et permet aux corps vertébraux de fusionner. Des exemples de cages de fusion intersomatique sont décrits dans les demandes de brevet internationales WO 02/089712 et WO 2005/055869.

Il existe plusieurs techniques de mise en place d'une cage de fusion intersomatique. La technique à laquelle s'intéresse tout particulièrement l'invention est la technique « TLIF » (Transforaminal Lumbar Interbody Fusion) selon laquelle la cage est introduite par une voie postérieure unilatérale ouverte à travers les tissus.

Une cage de fusion intersomatique doit être placée dans une position bien déterminée dans l'espace du disque intervertébral. La cage est plus précisément placée dans la partie antérieure dudit espace. Les cages de fusion intersomatique que l'on utilise dans le cadre de la technique TLIF ont généralement une forme arquée, avec une face convexe et une face concave. La face convexe doit être mise en regard du côté antérieur de l'espace du disque intervertébral et la face concave en regard du côté postérieur. Pour ce faire, il est nécessaire de faire tourner la cage après son introduction dans l'espace du disque intervertébral. Cette mise en place de la cage est assez délicate à réaliser et nécessite l'emploi d'un nombre relativement élevé d'instruments coudés.

La demande de brevet français FR 2 861 582 décrit une cage de fusion intersomatique et un instrument de préhension associé qui sont conçus pour assurer une liberté en rotation de la cage par rapport à l'instrument. A cet effet, la cage comporte à chacune de ses extrémités des évidements verticaux à section oblongue ménagés sur toute la hauteur de la cage et des encoches horizontales découchant dans les évidements verticaux. L'instrument comporte, lui, une tige dont l'extrémité est munie d'un ergot d'accrochage cylindrique qui peut être accouplé aux évidements verticaux à la manière d'une baïonnette. La tige est à l'intérieur d'un tube mobile, dit de blocage, qui peut être approché ou éloigné de l'ergot d'accrochage au moyen d'une molette pour serrer la paroi de la cage entre l'extrémité du tube et l'ergot d'accrochage, et ainsi solidariser temporairement la cage et l'instrument, ou pour libérer cette paroi afin de permettre une rotation de l'instrument par rapport à la cage.

En autorisant une rotation entre la cage et son instrument de préhension, le nombre d'instruments nécessaires à la mise en place de la cage est réduit. Toutefois, malgré cette liberté en rotation, faire tourner la cage pour la mettre dans sa position finale reste une opération délicate. De plus, le mode de liaison entre la cage et l'instrument de préhension présente plusieurs inconvénients. Du fait de la section oblongue des évidements verticaux, un jeu en translation horizontale existera entre l'ergot d'accrochage et les évidements verticaux si le tube de blocage n'est pas suffisamment serré contre la paroi de la cage. Un tel jeu nuira à la précision de déplacement de la cage. Un autre inconvénient est que ce mode de liaison impose au chirurgien d'effectuer des serrages et desserrages successifs et fastidieux du tube de blocage pendant la mise en place de la cage.

La demande de brevet internationale WO 2005/087143 décrit un implant intervertébral adapté à une introduction par voie postérieure unilatérale. Cet implant comporte sur ses faces supérieure et inférieure des rainures et des arêtes longitudinales de guidage.

La demande de brevet internationale WO 2006/079356 décrit également un implant intervertébral adapté à une introduction par voie postérieure unilatérale. Cet implant comporte à l'une de ses extrémités des moyens d'accouplement destinés à coopérer avec un instrument de positionnement de l'implant entre deux corps vertébraux. Ces moyens d'accouplement comportent un axe disposé dans un évidement d'extrémité et muni d'un trou destiné à recevoir la pointe de l'instrument de positionnement. La course de l'instrument de positionnement est contenu dans un plan parallèle aux faces supérieure et inférieure de l'implant et limitée par l'ouverture de la fenêtre de l'évidement.

Un autre implant intervertébral adapté à une introduction par voie postérieure unilatérale est décrit dans la demande de brevet allemand DE 19710392. Ledit implant comporte un ensemble d'éléments en forme de secteurs annulaires qui sont articulés au niveau de leurs faces latérales et reliés par des fils qui les traversent. Ces éléments sont introduits entre les deux corps vertébraux en étant sensiblement alignés puis sont rapprochés les uns des autres en étant guidés par les fils de manière à former ensemble un implant annulaire.

La présente invention vise à proposer un implant intervertébral conçu pour faciliter sa rotation dans sa position finale lors de sa mise en place entre deux corps vertébraux.

A cette fin, il est prévu un implant intervertébral comprenant un corps comprenant des faces supérieure et inférieure destinées à être en contact avec des corps vertébraux respectifs pour maintenir un espace souhaité entre ces corps vertébraux, caractérisé en ce qu'il comprend un pivot sur l'une au moins des faces supérieure et inférieure. Un tel pivot peut coopérer avec le corps vertébral correspondant pendant la mise en place de l'implant pour permettre une rotation aisée de celui-ci autour de l'axe imaginaire défini par le pivot.

Pour faciliter encore sa mise en place, l'implant intervertébral selon l'invention comprend en outre, de préférence, des moyens d'accouplement à un instrument de préhension agencés pour autoriser une rotation de l'instrument par rapport à l'implant, ces moyens d'accouplement comprenant un logement ménagé dans le corps et débouchant sur une face périphérique dudit corps et un organe mobile en rotation dans le logement, cet organe mobile étant apte à s'accoupler avec une extrémité de l'instrument. De tels moyens d'accouplement permettent l'utilisation d'un instrument de préhension sans tube de blocage et simple à manipuler.

De préférence, afin de faciliter encore la mise en place de l'implant selon l'invention, le corps comprend des première et seconde parties articulées horizontalement entre une première position relative où les première et seconde parties sont sensiblement alignées et une seconde position relative où les première et seconde parties font un angle entre elles. La première position relative confère à l'implant une forme temporaire droite qui lui permet de se déplacer plus aisément dans le passage ou voie postérieur, généralement étroit, qui est pratiqué dans les tissus. La seconde position relative est la position finale, qui donne à l'implant une forme arquée adaptée à sa fonction.

Des modes de réalisation particuliers de l'invention sont définis dans les revendications dépendantes annexées.

D'autres caractéristiques et avantages de la présente invention apparaîtront à la lecture de la description détaillée suivante faite en référence aux dessins annexés dans lesquels :
- les figures 1 à 3 sont respectivement une vue en perspective de dessus, une vue de côté et une coupe horizontale en perspective, prise suivant le plan III-III de la figure 2, d'un implant intervertébral selon un premier mode de réalisation de l'invention ;
- les figures 4 et 5 sont respectivement une vue en perspective de dessus et une vue de côté d'un implant intervertébral selon un second mode de réalisation de l'invention ;
- les figures 6 à 8 sont respectivement une vue en perspective de dessus, une coupe horizontale en perspective et une vue plane de dessus d'un implant intervertébral selon un troisième mode de réalisation de l'invention ;
- les figures 9 et 10 sont respectivement des vues en perspective de deux parties formant le corps de l'implant intervertébral selon le troisième mode de réalisation de l'invention ;
- la figure 11 est une vue plane en coupe horizontale de l'implant intervertébral selon le troisième mode de réalisation de l'invention accouplé à deux instruments de préhension et de manipulation ;
- la figure 12 montre l'implant intervertébral selon le troisième mode de réalisation de l'invention dans sa position finale sur une vertèbre.

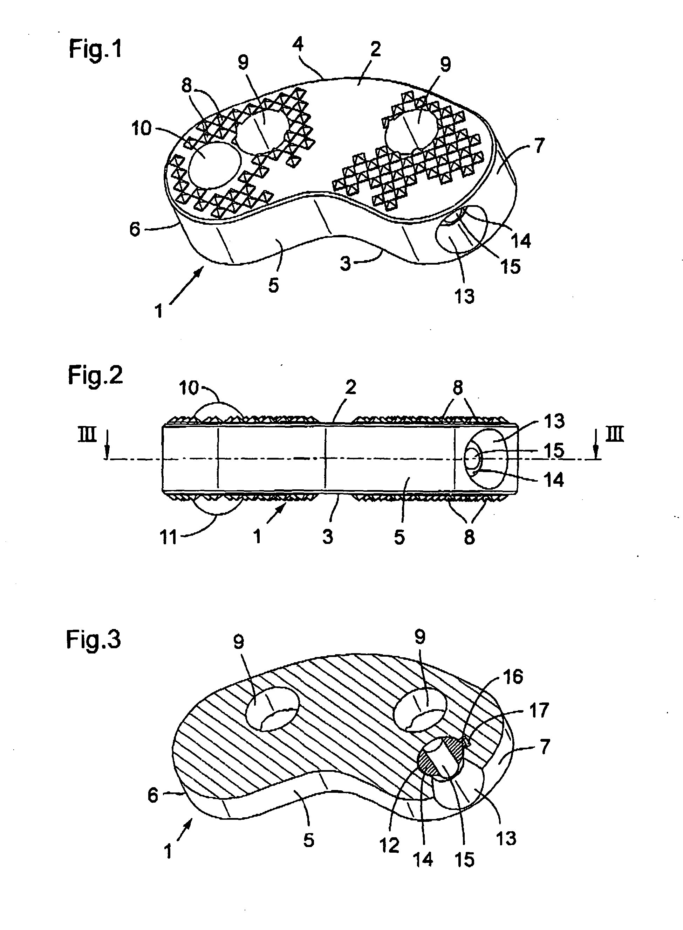

En référence aux figures 1 à 3, un implant intervertébral ou cage de fusion intersomatique selon un premier mode de réalisation de l'invention comprend un corps de forme arquée 1 comprenant une face supérieure 2, une face inférieure 3, une face antérieure convexe 4, une face postérieure concave 5 et deux faces d'extrémité arrondies 6, 7 reliant les faces antérieure 4 et postérieure 5. Les faces antérieure 4, postérieure 5 et d'extrémité 6, 7 définissent ensemble la périphérie du corps 1.

Les faces supérieure et inférieure 2, 3 sont destinées à être en contact avec deux corps vertébraux adjacents respectifs, plus précisément avec deux plateaux vertébraux. Des picots 8 sont prévus sur ces faces supérieure et inférieure 2, 3 pour s'ancrer dans les plateaux vertébraux.

Des orifices 9 traversant le corps 1 sur toute sa hauteur permettent la fusion des corps vertébraux. Des trous de vascularisation (non représentés) peuvent également être prévus.

Conformément à l'invention, le corps 1 comporte sur chacune des faces supérieure et inférieure 2, 3, ou au moins sur l'une d'entre elles, un pivot 10, 11. Les pivots 10, 11 sont situés à une extrémité du corps 1 et sont alignés l'un par rapport à l'autre. Dans l'exemple représenté, les pivots 10, 11 ont une forme hémisphérique. Ils pourraient néanmoins avoir une autre forme, par exemple conique ou pyramidale.

La cage peut être mise en place selon la technique TLIF. Une voie d'abord unilatérale postérieure est ouverte à droite ou à gauche de la colonne vertébrale, les corps vertébraux entre lesquels doit être introduite la cage sont distractés, les parties du disque intervertébral affectées sont retirées et la cage est introduite par ladite voie. La distraction des corps vertébraux doit être suffisante pour que les pivots 10, 11 ne puissent pas gêner l'introduction de la cage. Une fois la cage introduite dans l'espace du disque intervertébral, la distraction des corps vertébraux est relâchée, Les plateaux vertébraux viennent alors au contact des pivots 10, 11, qui forment une dépression dans les plateaux vertébraux. La cage peut ensuite être tournée aisément jusque dans sa position finale autour d'un axe imaginaire unique passant par les pivots 10, 11. Un impactage des corps vertébraux sur la cage permet aux plateaux vertébraux de venir au contact des faces supérieure et inférieure 2, 3 et aux picots 8 de s'ancrer dans les plateaux vertébraux pour fixer la cage en position.

Selon une autre caractéristique de l'invention, le corps 1 comprend à son extrémité opposée à celle comprenant les pivots 10, 11 un logement 12 débouchant sur sa périphérie, plus précisément sur la face d'extrémité 7. Dans l'exemple illustré, le logement 12 est sphérique et débouche sur la face d'extrémité 7 par l'intermédiaire d'une ouverture tronconique 13 évasée vers l'extérieur. Un organe d'accouplement de forme sphérique 14 est disposé dans le logement 12 de manière à pouvoir se déplacer en rotation sur lui-même, sans jeu, à la manière d'une rotule, tout en restant prisonnier du logement 12. Cet organe d'accouplement 14 est traversé le long d'un de ses axes par un alésage taraudé 15 dont le diamètre est plus petit que le petit diamètre de l'ouverture tronconique 13 et qui communique avec l'ouverture tronconique 13 pour pouvoir recevoir à travers cette ouverture 13 une extrémité filetée d'une tige d'un instrument de préhension (non représenté).

L'organe d'accouplement mobile 14 confère ainsi à la cage une liberté en rotation par rapport à son instrument de préhension dans des limites définies par la paroi de l'ouverture tronconique 13, paroi qui sert de surface d'appui et de butée pour l'instrument de préhension. Cette liberté en rotation s'exerce dans toutes les directions à l'intérieur du cône défini par la paroi de l'ouverture 13, et donc en particulier dans des plans horizontaux et verticaux. Un tel instrument de préhension pourra donc servir à la fois à faire entrer la cage dans l'espace du disque intervertébral et à faire tourner cette cage horizontalement pour lui donner sa position finale. La liberté en rotation verticale de la cage par rapport à l'instrument facilitera quant à elle l'introduction et le déplacement de la cage dans le passage la conduisant dans l'espace du disque intervertébral, passage qui est étroit et qui offre peu de visibilité au chirurgien. En variante, néanmoins, on peut donner à l'organe d'accouplement mobile 14 une forme cylindrique, plutôt que sphérique, si une telle liberté en rotation verticale n'est pas souhaitée.

Lorsque, comme dans l'exemple illustré, l'organe d'accouplement 14 a une forme sphérique, un ergot 16 situé sur l'organe d'accouplement 14 coopère avec un logement 17 de plus grande taille pratiqué dans le corps 1 pour bloquer en rotation l'organe d'accouplement 14 dans les sens du vissage et du dévissage de l'extrémité filetée de l'instrument dans l'alésage 15 et permettre ainsi l'accouplement et le désaccouplement de la cage et de l'instrument.

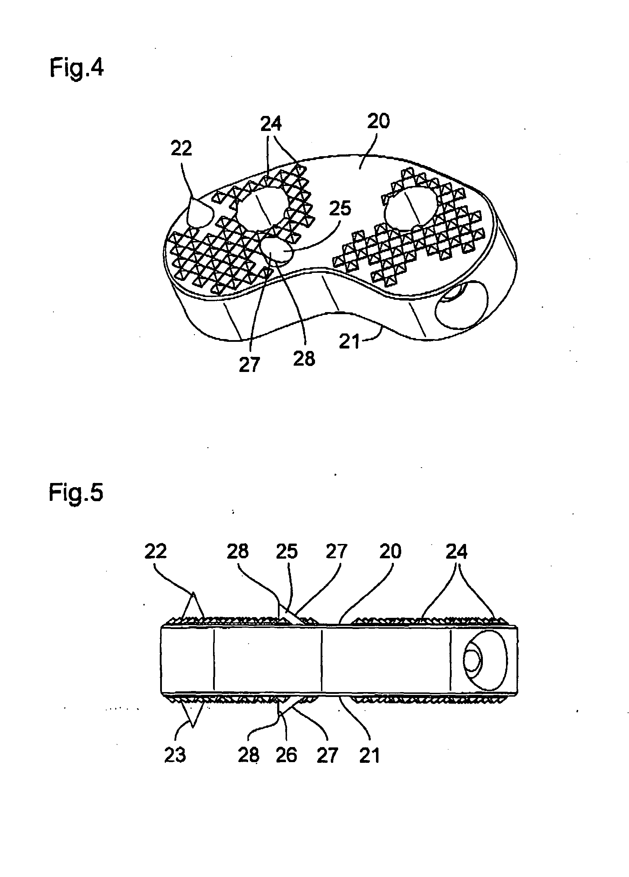

Les figures 4 et 5 montrent un implant intervertébral ou cage de fusion intersomatique selon un second mode de réalisation de l'invention. Dans ce second mode de réalisation, la cage comporte sur chacune ou l'une au moins de ses faces supérieure et inférieure 20, 21, en plus du pivot 22, 23, ici de forme conique, et des picots d'ancrage 24, une protubérance 25, 26 de hauteur inférieure à celle du pivot 22, 23 mais très supérieure à celle des picots d'ancrage 24. Les protubérances 25, 26 ont une face plane inclinée 27 qui définit, avec une face périphérique des protubérances 25, 26, une arête coupante 28. Cette arrête 28 a, dans le plan de la face 27, une forme courbe, de préférence elliptique comme représenté, permettant aux protubérances 25, 26 de venir s'ancrer progressivement dans les plateaux vertébraux pendant la manoeuvre de rotation de la cage autour de l'axe imaginaire passant par les pivots 22, 23 et le raprochement des vertèbres consécutif au relâchement de la distraction. Cet ancrage des protubérances 25, 26 permet de fixer la position finale de la cage avant l'impactage.

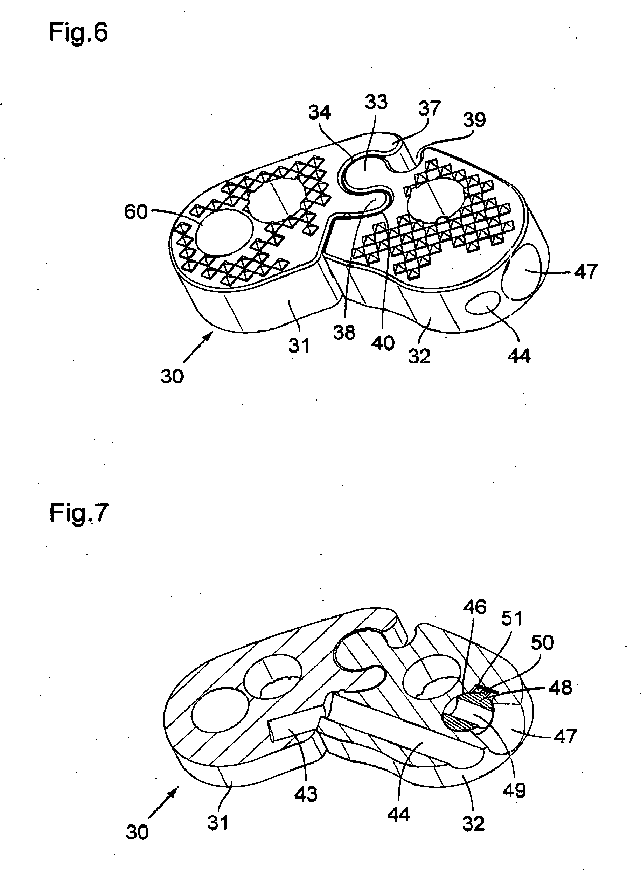

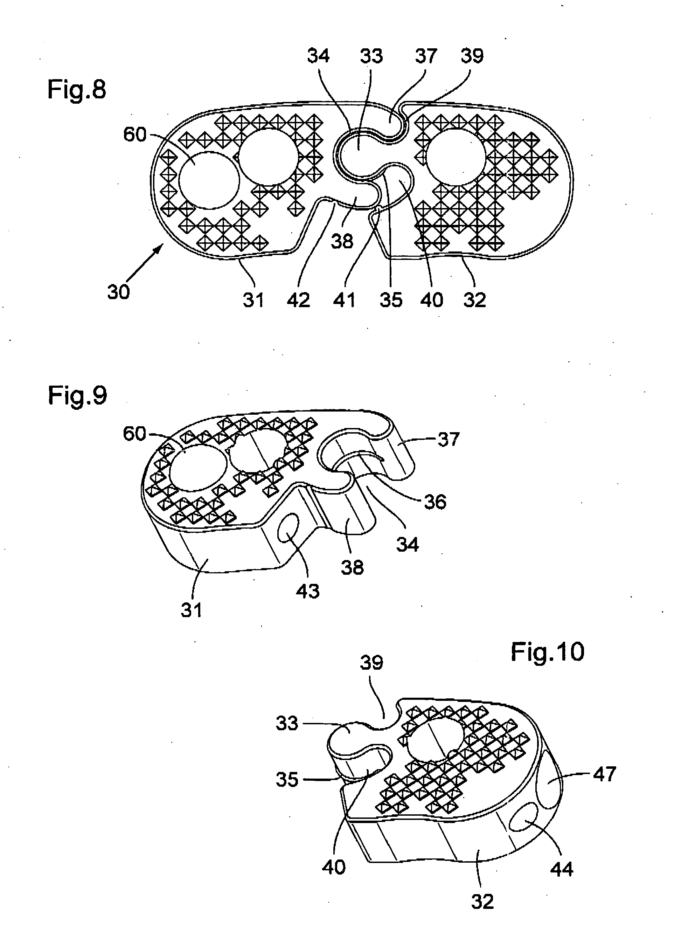

Les figures 6 à 11 montrent un implant intervertébral ou cage de fusion intersomatique selon un troisième mode de réalisation de l'invention. Dans ce troisième mode de réalisation, le corps 30 est constitué de deux parties 31, 32 articulées horizontalement l'une à l'autre entre une première position relative, dite position ouverte, où les parties 31, 32 sont alignées (figure 8) et une seconde position relative, dite position fermée, où les parties 31, 32 font un angle entre elles (figure 6) et confèrent à la cage une forme conventionnelle arquée.

L'articulation entre les parties 31, 32 est réalisée en faisant coopérer un organe d'articulation mâle 33, de forme cylindrique, de la partie 32 avec un organe d'articulation femelle ou cavité 34, de forme correspondante cylindrique, de la partie 31. L'organe mâle 33 comprend une nervure horizontale 35 le long de sa périphérie, nervure qui coopère avec une gorge horizontale correspondante 36 pratiquée dans la paroi de la cavité 34 pour positionner et bloquer verticalement les parties de corps 31, 32 l'une par rapport à l'autre. En variante, bien entendu, la nervure pourrait être située sur la paroi de la cavité 34 et la gorge correspondante dans l'organe mâle 33.

La cavité 34 définit latéralement deux organes de guidage et de butée 37, 38 qui coopèrent avec des cavités correspondantes 39, 40 de la partie 32 situées de part et d'autre de l'organe d'articulation mâle 33 pour guider et limiter la course relative des parties 31, 32. L'organe de guidage et de butée 37, respectivement 38, est en butée contre le fond de la cavité 39, respectivement 40, lorsque les parties 31, 32 sont dans leur position relative ouverte, respectivement fermée.

Celle des cavités 39, 40 la plus proche du côté postérieur de la cage, à savoir la cavité 40, comporte sur sa paroi latérale opposée à l'organe mate 33 une nervure verticale d'encliquetage 41 (cf. figure 8). Cette nervure verticale 41 glisse sur l'organe de guidage et de butée 38 en maintenant les parois latérales de la cavité 40 écartées élastiquement lorsque les parties 31, 32 se déplacent entre leur position relative ouverte et leur position relative fermée. La nervure 41 vient se loger dans une rainure verticale d'encliquetage correspondante 42 pratiquée dans l'organe de guidage et de butée 38 lorsque ce dernier arrive en butée dans le fond de la cavité 40, c'est-à-dire lorsque les parties 31, 32 atteignent leur position relative fermée. Dans cette position, la cavité 40 retrouve sa forme de repos, non déformée, et les parties de corps 31, 32 sont verrouillées. En variante, bien entendu, la nervure verticale 41 pourrait être située sur l'organe de guidage et de butée 38 et la rainure correspondante 42 dans la paroi de la cavité 40.

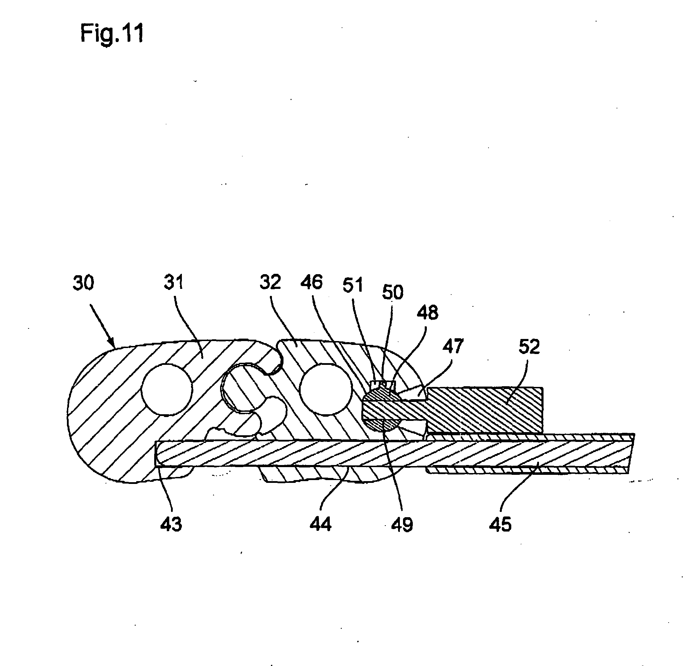

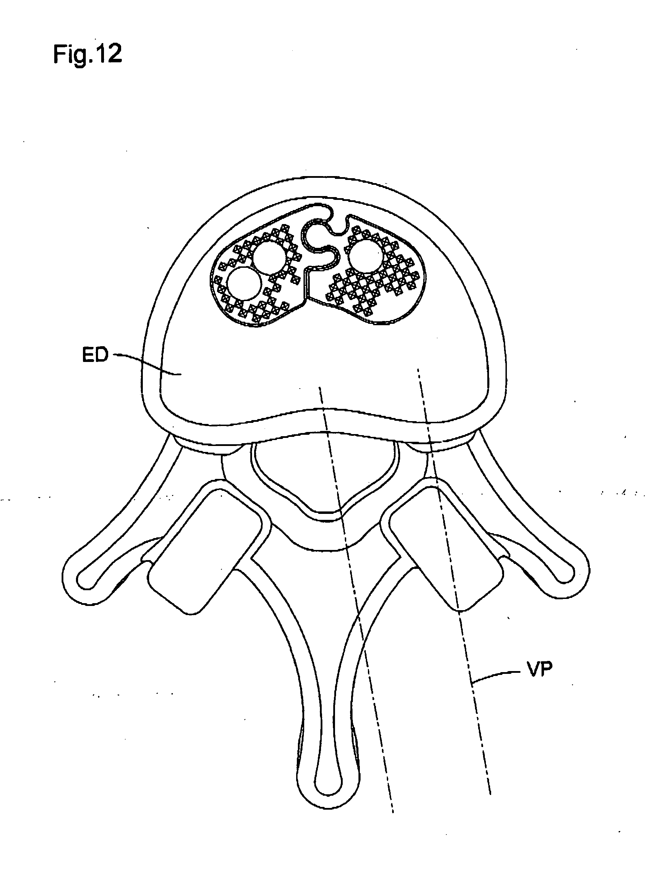

La position relative fermée des parties de corps 31, 32 est particulièrement adaptée à l'introduction de la cage dans l'espace du disque intervertébral. En effet, comme déjà indiqué, le passage ou voie postérieur pratiqué à travers les tissus par lequel la cage est introduite est étroit. A titre d'illustration, ce passage a été représenté de manière schématique à la figure 12 par des traits pointillés désignés par le repère VP, l'espace du disque intervertébral étant lui désigné par le repère ED. La forme conventionnelle arquée d'une cage de fusion intersomatique ne facilite pas l'introduction et le déplacement de la cage dans ce passage VP. La cage articulée selon ce troisième mode de réalisation de l'invention est introduite et déplacée longitudinalement dans ce passage VP alors que les parties de corps 31, 32 sont dans leur position relative ouverte, où la cage est rectiligne (figure 8). A cet effet, les parties de corps 31, 32 comprennent dans leur épaisseur respectivement un trou longitudinal borgne 43 et un trou longitudinal traversant 44 qui sont alignés lorsque les parties de corps 31, 32 sont dans leur position relative ouverte. Ces trous 43, 44 sont destinés à recevoir une tige 45 d'un premier instrument de préhension (figure 11), qui maintient les parties de corps 31, 32 dans leur position relative ouverte. Cet instrument est retiré progressivement dès que la cage a pénétré dans l'espace du disque intervertébral ED.

La position relative ouverte des parties de corps 31, 32 correspond, quant à elle, à la position d'implantation finale de la cage, comme montré à la figure 12. Pour pouvoir manipuler la cage et la mettre dans sa position finale après qu'elle a pénétré dans l'espace du disque intervertébral ED, la cage selon ce troisième mode de réalisation comprend des moyens d'accouplement 46 à 51 identiques aux moyens 12 à 17 du premier mode de réalisation. Ces moyens d'accouplement 46 à 51 reçoivent un second instrument de préhension, désigné à la figure 11 par le repère 52, qui, grâce à sa liberté en rotation par rapport à la cage, permet de faire tourner la cage dans son ensemble pour la placer dans la partie antérieure de l'espace du disque intervertébral ED et de faire tourner les parties de corps 31, 32 l'une par rapport à l'autre jusqu'au verrouillage de ces parties 31, 32 dans leur position relative fermée. Comme le premier instrument 45, ce second instrument 52 est accouplé à la cage avant son introduction dans le passage VP. Il est devissé de la cage et retiré dès que la cage a été mise dans sa position finale.

La cage de fusion intersomatique selon ce troisième mode de réalisation peut aussi comprendre un pivot 50 et une protubérance coupante correspondante (non représentée) sur l'une ou chacune des faces supérieure et inférieure du corps 30, comme dans le premier mode de réalisation, pour faciliter la rotation puis le positionnement de la cage.

## Revendications

1. Implant intervertébral comprenant un corps (1) comprenant des faces supérieure et inférieure (2, 3) destinées à être en contact avec des corps vertébraux respectifs pour maintenir un espace souhaité entre ces corps vertébraux, **caractérisé en ce qu'**il comprend un pivot (10, 11) sur l'une au moins des faces supérieure et inférieure (2, 3) pour faciliter une rotation de l'implant lors de sa mise en place, ladite rotation s'effectuant autour d'un axe unique passant par le(s)dit(s) pivot(s) (10,11).

2. Implant intervertébral selon la revendication 1, **caractérisé en ce qu'**il comprend, respectivement sur la face supérieure (2) et la face inférieure (3), des premier et second pivots (10, 11) alignés.

3. Implant intervertébral selon la revendication 1 ou 2, **caractérisé en ce que** le ou les pivots (10, 11) sont situés à une extrémité de l'implant.

4. Implant intervertébral selon l'une quelconque des revendications 1 à 3, **caractérisé en ce qu'**il comprend en outre, sur la ou l'une au moins des faces (2, 3) comprenant un pivot (10, 11), une protubérance (25, 26) définissant une arête coupante (28), cette arête coupante (28) étant située dans un plan incliné (27) et présentant dans ce plan une forme courbe.

5. Implant intervertébral selon la revendication 4, **caractérisé en ce qu'**il comprend en outre, sur la ou l'une au moins des faces (2, 3) comprenant un pivot (10, 11) et une protubérance (25, 26), des éléments d'ancrage (8) de hauteur inférieure à celle de la protubérance correspondante (25, 26).

6. Implant intervertébral selon l'une quelconque des revendications 1 à 5, **caractérisé en ce qu'**il comprend en outre des moyens (12, 13, 14) d'accouplement à un instrument de préhension agencés pour autoriser une rotation de l'instrument par rapport à l'implant, ces moyens d'accouplement comprenant :
- un logement (12) ménagé dans le corps (1) et débouchant sur une face périphérique (7) dudit corps (1), et
- un organe d'accouplement (14) mobile en rotation dans le logement (12), cet organe d'accouplement (14) étant apte à s'accoupler avec une extrémité de l'instrument.

7. Implant intervertébral selon la revendication 6, **caractérisé en ce que** l'organe d'accouplement (14) est disposé dans le logement (12) sensiblement sans jeu en translation.

8. Implant intervertébral selon la revendication 6 ou 7, **caractérisé en ce que** l'organe d'accouplement (14) comprend un trou taraudé (15) apte à recevoir une extrémité filetée de l'instrument.

9. Implant intervertébral selon l'une quelconque des revendications 6 à 8, **caractérisé en ce que** l'organe d'accouplement (14) est mobile au moins en rotation horizontale et en rotation verticale, permettant à l'instrument d'être déplacé au moins en rotation horizontale et en rotation verticale par rapport au corps (1).

10. Implant intervertébral selon l'une quelconque des revendications 6 à 9,
**caractérisé en ce que** le logement (12) et l'organe d'accouplement (14) ont une forme sensiblement sphérique.

11. Implant intervertébral selon l'une quelconque des revendications 6 à 10, **caractérisé en ce que** le logement (12) débouche sur la face périphérique (7) du corps (1) par l'intermédiaire d'une ouverture (13) dont la paroi limite les mouvements en rotation de l'instrument par rapport au corps (1).

12. Implant intervertébral selon l'une quelconque des revendications 1 à 11,
**caractérisé en ce que** le corps (30) comprend des première et seconde parties (31, 32) articulées horizontalement entre une première position relative où les première et seconde parties (31, 32) sont sensiblement alignées et une seconde position relative où les première et seconde parties (31, 32) font un angle entre elles.

13. Implant intervertébral selon la revendication 12, **caractérisé en ce qu'**il comprend des moyens (41, 42) pour verrouiller les première et seconde parties (31, 32) du corps (30) dans leur seconde position relative.

14. Implant intervertébral selon la revendication 13, **caractérisé en ce que** les moyens de verrouillage (41, 42) sont des moyens d'encliquetage.

15. Implant intervertébral selon l'une quelconque des revendications 12 à 14, **caractérisé en ce que** l'une des première et seconde parties (31, 32) du corps (30), dite partie mâle (32), comprend un organe d'articulation mâle (33) coopérant avec un organe d'articulation femelle (34) de l'autre partie du corps (30), dite partie femelle (31), pour l'articulation des première et seconde parties (31, 32), et **en ce que** l'organe d'articulation femelle (34) définit des premier et second organes de guidage et de butée (37, 38) qui coopèrent avec des première et seconde cavités (39, 40) de la partie mâle (32) lorsque lesdites parties mâle et femelle (31, 32) sont dans leurs première et seconde positions relatives, respectivement.

16. Implant intervertébral selon les revendications 14 et 15, **caractérisé en ce que** les moyens d'encliquetage comprennent une saillie (41) située sur la paroi de la seconde cavité (40) ou sur le second organe de guidage et de butée (38) et un évidement correspondant (42) pratiqué dans le second organe de guidage et de butée (38) ou dans la paroi de la seconde cavité (40).

17. Implant intervertébral selon la revendication 15 ou 16, **caractérisé en ce que** les organes d'articulation mâle et femelle (33, 34) ont une forme sensiblement cylindrique et comprennent l'un une nervure horizontale (35) et l'autre une gorge horizontale correspondante (36) qui coopèrent pour bloquer verticalement les première et seconde parties (31, 32) du corps (30) l'une par rapport à l'autre.

18. Implant intervertébral selon l'une quelconque des revendications 12 à 17, **caractérisé en ce que** les première et seconde parties (31, 32) du corps (30) comprennent respectivement des premier et second trous longitudinaux (43, 44) qui sont alignés lorsque les première et seconde parties (31, 32) du corps (30) sont dans leur première position relative de sorte à pouvoir recevoir une tige (45) d'un instrument pour maintenir les parties (31, 32) du corps (30) dans ladite première position relative.

19. Implant intervertébral selon l'une quelconque des revendications 1 à 18, **caractérisé en ce que** sa forme et sa taille sont adaptées à une introduction entre deux corps vertébraux par voie postérieure unilatérale.

## Claims

1. Intervertebral implant comprising a body (1) comprising an upper and a lower face (2, 3) intended to be in contact with respective vertebral bodies to maintain a desired space between these vertebral bodies, **characterized in that** it comprises a pivot (10, 11) on at least one of the upper and lower faces (2, 3) to ease a rotation of the implant during its positioning, where the said rotation is carried out round a unique axis passing through the said pivot(s) (10,11).

2. Intervertebral implant according to claim 1, **characterized in that** it comprises on respectively the upper face (2) and the lower face (3) a first and a second pivot (10, 11), which are aligned.

3. Intervertebral implant according to claim 1 or 2, **characterized in that** the pivot(s) (10, 11) is located at one end of the implant.

4. Intervertebral implant according to any of claims 1 to 3, **characterized in that** it further comprises, on the face or at least one of the faces (2, 3) comprising a pivot (10, 11), a protuberance (25, 26) defining a cutting edge (28), the said cutting edge (28) being located in an inclined plane (27) and presenting a curved shape in said plane.

5. Intervertebral implant according to claim 4, **characterized in that** it further comprises, on the face or at least one of the faces (2, 3) comprising a pivot (10, 11) and a protuberance (25, 26), anchoring elements (8) the height of which is less than the height of the corresponding protuberance (25, 26).

6. Intervertebral implant according to any of claims 1 to 5, **characterized in that** it further comprises means (12, 13, 14) for coupling to a gripping instrument, the said coupling means being arranged to allow a rotation of the instrument with respect to the implant, the said coupling means comprising:
■ a recess (12) made in the body (1) and opening on a peripheral face (7) of the said body (1), and
■ a coupling organ (14) mobile in rotation in the recess (12), the said coupling organ (14) being adapted to couple with an end of the instrument.

7. Intervertebral implant according to claim 6, **characterized in that** the coupling organ (14) is set in the recess (12) substantially without play in translation.

8. Intervertebral implant according to claim 6 or 7, **characterized in that** the coupling organ (14) comprises a threaded hole (15) designed to receive a threaded end of the instrument.

9. Intervertebral implant according to any one of claims 6 to 8, **characterized in that** the coupling organ (14) is mobile at least in horizontal rotation and in vertical rotation, allowing the instrument to be moved at least in horizontal rotation and in vertical rotation with respect to the body (1).

10. Intervertebral implant according to any of claims 6 to 9, **characterized in that** the recess (12) and the coupling organ (14) have a substantially spherical shape.

11. Intervertebral implant according to any of claims 6 to 10,
**characterized in that** the recess (12) opens on the peripheral face (7) of the body (1) through an opening (13) the inner surface of which limits the movements in rotation of the instrument with respect to the body (1).

12. Intervertebral implant according to any of claims 1 to 11,
**characterized in that** the body (30) comprises a first and a second part (31, 32) articulated horizontally between a first relative position where the first and second parts (31, 32) are substantially aligned and a second relative position where the first and second parts (31, 32) make an angle between them.

13. Intervertebral implant according to claim 12, **characterized in that** it comprises means (41, 42) for locking the first and second parts (31, 32) of the body (30) in their second relative position.

14. Intervertebral implant according to claim 13, **characterized in that** the locking means (31, 32) are detent means.

15. Intervertebral implant according to any of claims 12 to 14,
**characterized in that** one of the first and second parts (31, 32) of the body (30), called male part (32), comprises a male articulation organ (33) cooperating with a female articulation organ (34) of the other part of the body (30), called the female part (31), for the articulation of the first and the second parts (31, 32) and **in that** the female articulation organ (34) defines a first and a second guiding and stopping organ (37, 38) that cooperate with a first and a second cavity (39, 40) of the male part (32) when the said male and female parts (32, 31) are respectively in their first and second relative positions.

16. Intervertebral implant according to claims 14 and 15, **characterized in that** the detent means comprise a protrusion (41) located on the inner surface of the second cavity (40) or on the second guiding and stopping organ (38) and a corresponding recess (42) located in the second guiding and stopping organ (38) or in the inner surface of the second cavity (40).

17. Intervertebral implant according to claim 15 or 16, **characterized in that** the male and female articulation organs (33, 34) have a substantially cylindrical shape and comprise for one a horizontal rib (35) and for the other a corresponding horizontal groove (36) that cooperate to vertically block the first and second parts (31, 32) of the body (30) with respect to each other.

18. Intervertebral implant according to any of claims 12 to 17,
**characterized in that** the first and second parts (31, 32) of the body (30) comprise respectively a first and a second longitudinal hole (43, 44) that are aligned when the first and second parts (31, 32) of the body (30) are in their first relative position in order to be able to receive a rod (45) of an instrument to maintain these parts (31, 32) of the body (30) in the said first relative position.

19. Intervertebral implant according to any of claims 1 to 18,
**characterized in that** the shape and the size of the implant are adapted for the implant to be inserted between two vertebral bodies through unilateral posterior way.

## Patentansprüche

1. Zwischenwirbelimplantat, welches einen Körper (1) umfasst, der eine obere und untere (2, 3) Fläche umfasst, welche dazu bestimmt sind, in Kontakt mit entsprechenden Wirbelkörpern zu stehen, um einen gewünschten Zwischenraum zwischen diesen Wirbelkörpern beizubehalten, **dadurch gekennzeichnet, dass** es einen Drehpunkt (10, 11) auf zumindest einer der beiden, nämlich der oberen und der unteren (2, 3), Flächen umfasst, um eine Rotation des Implantats zu ermöglichen, wenn dieses eingesetzt wird, wobei die genannte Rotation um eine einzige Achse erfolgt, welche durch den/die genannten Drehpunkt (10, 11) verläuft.

2. Zwischenwirbelimplantat nach Anspruch 1, **dadurch gekennzeichnet, dass** es auf der oberen Fläche (2) und der unteren Fläche (3) einen ersten bzw. zweiten Drehpunkt (10, 11) umfasst, welche miteinander fluchten sind.

3. Zwischenwirbelimplantat nach Anspruch 1 oder 2, **dadurch gekennzeichnet, dass** sich der oder die Drehpunkte (10, 11) an einem Ende des Implantats befinden.

4. Zwischenwirbelimplantat nach einem beliebigen der Ansprüche 1 bis 3, **dadurch gekennzeichnet, dass** es zudem auf der oder zumindest einer der Flächen (2, 3), die einen Drehpunkt (10, 11) umfassen, einen Vorsprung (25, 26) umfasst, der eine scharfe Kante (28) bildet, wobei sich diese scharfe Kante (28) in einer schrägen Ebene (27) befindet und in dieser Ebene eine gebogene Form aufweist.

5. Zwischenwirbelimplantat nach Anspruch 4, **dadurch gekennzeichnet, dass** es zudem auf der oder zumindest einer der Flächen (2, 3), die einen Drehpunkt (10, 11) und einen Vorsprung (25, 26) umfassen, Verankerungselemente (8) umfasst, deren Höhe geringer Ist als die des entsprechenden Vorsprungs (25, 26).

6. Zwischenwirbelimplantat nach einem beliebigen der Ansprüche 1 bis 5, **dadurch gekennzeichnet, dass** es zudem Mittel (12, 13, 14) zur Kopplung an ein Greifinstrument umfasst, welche angeordnet sind, um eine Rotation des Instrumentes in Bezug auf das Implantat zuzulassen, wobei diese Kopplungsmittel Folgendes umfassen:
- eine Aufnahme (12), welche in dem Körper (1) angebracht ist und in eine Umfangsfläche (7) des genannten Körpers (1) mündet, und
- ein Kopplungsorgan (14), beweglich in Rotation in der Aufnahme (12), wobei dieses Kopplungsorgan (14) geeignet ist, mit einem Ende des Instrumentes gekoppelt zu werden.

7. Zwischenwirbelimplantat nach Anspruch 6, **dadurch gekennzeichnet, dass** das Kopplungsorgan (14) in der Aufnahme (12) im Wesentlichen ohne Spiel bei der Translation angeordnet ist.

8. Zwischenwirbelimplantat nach Anspruch 6 oder 7, **dadurch gekennzeichnet, dass** das Kopplungsorgan (14) eine Gewindebohrung (15) umfasst, welche geeignet ist, ein mit einem Gewinde versehenes Ende des Instrumentes aufzunehmen.

9. Zwischenwirbelimplantat nach einem beliebigen der Ansprüche 6 bis 8, **dadurch gekennzeichnet, dass** das Kopplungsorgan (14) zumindest in horizontaler Rotation und in vertikaler Rotation beweglich ist, was ermöglicht, dass das Instrument zumindest in horizontaler Rotation und in vertikaler Rotation in Bezug auf den Körper (1) verschoben wird.

10. Zwischenwirbelimplantat nach einem beliebigen der Ansprüche 6 bis 9, **dadurch gekennzeichnet, dass** die Aufnahme (12) und das Kopplungsorgan (14) eine im Wesentlichen sphärische Form aufweisen.

11. Zwischenwirbelimplantat nach einem beliebigen der Ansprüche 6 bis 10, **dadurch gekennzeichnet, dass** die Aufnahme (12) in die Umfangsfläche (7) des Körpers (1) durch eine Öffnung (13) mündet, deren Wand die Rotationsbewegungen des Instrumentes in Bezug auf den Körpers (1) begrenzt.

12. Zwlschenwirbelimplantat nach einem beliebigen der Ansprüche 1 bis 11, **dadurch gekennzeichnet, dass** der Körper (30) einen ersten und zweiten Teil (31, 32) umfasst, welche horizontal miteinander zwischen einer ersten relativen Position, in der der erste und zweite Teil (31, 32) im Wesentlichen miteinander fluchten, und einer zweiten relativen Position, in der der erste und zweite Teil (31, 32) einen Winkel miteinander bilden, gelenkig verbunden sind.

13. Zwischenwirbelimplantat nach Anspruch 12, **dadurch gekennzeichnet, dass** es Mittel (41, 42) umfasst, um den ersten und zweiten Teil (31, 32) des Körpers (30) in ihrer zweiten relativen Position zu verriegeln.

14. Zwischenwirbelimplantat nach Anspruch 13, **dadurch gekennzeichnet, dass** die Verriegelungsmittel (41, 42) Einrastmittel sind.

15. Zwischenwirbelimplantat nach einem beliebigen der Ansprüche 12 bis 14, **dadurch gekennzeichnet, dass** eines der Teile, nämlich des ersten und zweiten Teils (31, 32) des Körpers (30), männliches Teil (32) genannt, ein männliches Gelenkorgan (33) umfasst, welches mit einem weiblichen Gelenkorgan (34) des anderen Teils des Körpers (30), weibliches Teil (31) genannt, zur gelenkigen Verbindung des ersten und zweiten Teils (31, 32) zusammenwirkt und dass das weibliche Gelenkorgan (34) ein erstes und zweites Führungs- und Anschlagsorgan (37, 38) definiert, welche mit einer ersten und zweiten Aussparung (39, 40) des männlichen Teils (32) zusammenwirken, wenn sich das genannte männliche und das genannte weibliche Teil (31, 32) in ihrer ersten bzw. zweiten relativen Position befinden.

16. Zwischenwirbalimplantat nach den Ansprüchen 14 und 15, **dadurch gekennzeichnet, dass** die Einrastmittel eine Vorkragung (41), die sich an der Wand der zweiten Aussparung (40) oder an dem zweiten Führungs- und Anschlagsorgan (38) befindet, und eine entsprechende Ausnehmung (42) umfasst, die in dem zweiten Führungs- und Anschlagsmittel (38) oder in der Wand der zweiten Aussparung (40) angebracht ist.

17. Zwischenwirbelimplantat nach den Ansprüchen 15 oder 16, **dadurch gekennzeichnet, dass** das männliche und weibliche Gelenkmittel (33, 34) eine im Wesentlichen zylindrische Form aufweisen und eines eine horizontale Rippe (35) und das andere eine entsprechende horizontale Auskehlung (36) umfasst, welche zusammenwirken, um das erste und zweite Teil (31, 32) des Körpers (30) vertikal in Bezug zueinander zu blockieren.

18. Zwischenwirbelimplantat nach einem beliebigen der Ansprüche 12 bis 17, **dadurch gekennzeichnet, dass** das erste und zweite Teil (31, 32) des Körpers (30) eine erste bzw. zweite Längsbohrung (43, 44) umfasst, welche miteinander fluchten, wenn sich das erste und zweite Teil (31, 32) des Körpers (30) in ihrer ersten relativen Position befinden, um einen Schaft (45) eines Instrumentes aufnehmen zu können, um die Teile (31, 32) des Körpers (30) in der genannten ersten relativen Position zu halten.

19. Zwischenwlrbelimplantat nach einem beliebigen der Ansprüche 1 bis 18, **dadurch gekennzeichnet, dass** seine Form und seine Größe für eine Einführung zwischen zwei Wirbelkörpern auf unilateralem posteriorem Weg ausgelegt sind.
